# EUROPEAN PATENT SPECIFICATION

(11) **EP 3 545 311 B1**
(45) Date of publication and mention of the grant of the patent: **27.03.2024**
(21) Application number: 17821475.5
(22) Date of filing: 23.11.2017
(51) Int. Cl.: G01N 33/68, C07K 16/18

(54) **GFAP DERIVATIVES FOR STROKE DIAGNOSTICS**
GFAP-DERIVATE ZUR DIAGNOSE EINES SCHLAGANFALLS
DÉRIVÉS DE GFAP POUR DIAGNOSTICS D'ACCIDENT VASCULAIRE CÉRÉBRAL

(30) Priority: 23.11.2016 GB 201619820; 22.12.2016 GB 201621964
(43) Date of publication of application: 02.10.2019
(73) Proprietor: Randox Laboratories Ltd, Crumlin, Antrim BT29 4QY (GB); Randox Teoranta, Dungloe F94 TV06 (IE)
(72) Inventor: RICHARDSON, Ciaran, Dungloe F94 TV06 (IE); MCCONNELL, Ivan, Crumlin Antrim BT294Qy (GB); LAMONT, John, Crumlin Antrim BT294QY (GB); FITZGERALD, Stephen Peter, Crumlin Antrim BT294QY (GB)
(74) Representative: Gill Jennings & Every LLP
(86) International application number: PCT/EP2017/080247
(87) International publication number: WO 2018/096049

(56) References cited:
- CHANGHONG REN ET AL: "Assessment of Serum UCH-L1 and GFAP in Acute Stroke Patients", SCIENTIFIC REPORTS, vol. 6, no. 1, 14 April 2016 (2016-04-14), XP055454920, DOI: 10.1038/srep24588
- M. T. WUNDERLICH ET AL: "Release of glial fibrillary acidic protein is related to the neurovascular status in acute ischemic stroke", EUROPEAN JOURNAL OF NEUROLOGY, vol. 13, no. 10, 1 October 2006 (2006-10-01), pages 1118-1123, XP055454923, GB ISSN: 1351-5101, DOI: 10.1111/j.1468-1331.2006.01435.x
- CHRISTOPH A. MAYER ET AL: "Blood Levels of Glial Fibrillary Acidic Protein (GFAP) in Patients with Neurological Diseases", PLOS ONE, vol. 8, no. 4, 23 April 2013 (2013-04-23), page e62101, XP055454927, DOI: 10.1371/journal.pone.0062101
- MEI-HSUAN CHEN ET AL: "Caspase Cleavage of GFAP Produces an Assembly-Compromised Proteolytic Fragment that Promotes Filament Aggregation", ASN NEURO, vol. 5, no. 5, 11 November 2013 (2013-11-11), XP055454938, ISSN: 1759-0914, DOI: 10.1042/AN20130032
- LINDA PAPA ET AL: "Elevated Levels of Serum Glial Fibrillary Acidic Protein Breakdown Products in Mild and Moderate Traumatic Brain Injury Are Associated With Intracranial Lesions and Neurosurgical Intervention", ANNALS OF EMERGENCY MEDICINE, vol. 59, no. 6, 1 June 2012 (2012-06-01), pages 471-483, XP055051391, ISSN: 0196-0644, DOI: 10.1016/j.annemergmed.2011.08.021
- KIMIKAZU FUJITA ET AL: "Increase of glial fibrillary acidic protein fragments in the spinal cord of motor neuron degeneration mutant mouse", BRAIN RESEARCH, vol. 785, no. 1, 1 February 1998 (1998-02-01), pages 31-40, XP055454939, AMSTERDAM, NL ISSN: 0006-8993, DOI: 10.1016/S0006-8993(97)00612-4
- YOKO SUZUKI ET AL: "Identification of nitrated proteins in the normal rat brain using a proteomics approach", NEUROLOGICAL RESEARCH, vol. 27, no. 6, 19 September 2005 (2005-09-19), pages 630-633, XP055454935, GB ISSN: 0161-6412, DOI: 10.1179/016164105X22039

## Description

### BACKGROUND

Stroke is a leading cause of death worldwide and can be defined as the rapidly developing loss of brain function(s) due to interruption in the blood supply to the brain. According to the World Health Organisation, 15 million people per annum suffer stroke world-wide with 5 million dying and a further 5 million being permanently disabled. An ischaemic stroke (IS) results in the blood supply to the brain being decreased resulting in brain damage and occurs when a blood vessel becomes blocked, usually via a blood clot. This clot may form locally at an atherosclerotic plaque (thrombotic stroke) or alternatively may occur due to a travelling particle or debris that has originated from elsewhere in the bloodstream (embolic stroke). The transient ischaemic attack (TIA) occurs when blood supply to the brain is temporarily decreased. A TIA is diagnosed if symptoms are quickly resolved (within 24 hours with the individual returning to normal health. Haemorrhagic stroke (HS) is the accumulation of blood within the skull vault and can be divided into its two main sub-types, intracerebral haemorrhage (ICH) in which blood leaks into brain tissue, and subarachnoid haemorrhage (SAH) in which blood escapes to the subarachnoid space. A haemorrhagic stroke occurs when a weakened blood vessel ruptures. The stroke-related condition in which an individual presenting with IS transforms at a later time point to HS, is termed `Haemorrhagic transformation' (HT). Ischaemic stroke accounts for approximately 85 per cent of all stroke cases and haemorrhagic stroke 15 per cent. The 30day fatality rate for IS is 8-12% and HS is 37-38%. To minimise neurological damage and death following stroke it is crucial that stroke patients are rapidly and accurately diagnosed so that appropriate treatment can be administered. For example, to breakdown clots anti-thrombolytic therapy such as tissue plasminogen activator (tPA) can be administered. However, such therapy is only warranted in IS and is detrimental in HS; the nature of TIA does not require such therapy and blood thinners such as warfarin and aspirin are prescribed in such cases. HT can occur as part of the natural evolution of IS or as a result of anticoagulant or thrombolytic therapy in the acute phase of IS. This poses a serious problem to both the stroke-affected individual and the clinician with respect to decisions on anti-thrombolytic and anti-coagulative therapy use. Therefore, patients admitted to a clinic with stroke-like symptoms present the clinician with a highly complex differential diagnosis task. A clinician who upon confronting a patient suspects stroke must delineate the various stroke-like conditions - stroke mimics (hypoglycemia, drug overdose, migraine, seizures, hyponatremia, loss of consciousness, intracranial tumours, subdural hematoma, hypertensive encephalopathy, encephalitis/ meningitis), TIA, IS and strokes that transform into haemorrhagic stroke, and a wrong diagnosis could have fatal consequences. At present if stroke is suspected, physical symptoms are evaluated and a CT scan is usually performed. A CT scan has good sensitivity for identifying HS patients (approximately 90% sensitivity) but has poor sensitivity for the detection of IS. The detection of stroke-related biomolecules (biomarkers) in biological fluids are a potential supportive means of stroke diagnosis. Biomarkers have the potential to expedite and increase the accuracy of stroke diagnosis and various candidate biomarkers have been proposed for diagnosis of stroke. Speed of diagnosis of stroke is especially critical to ensure the best possible outcome for the patient as it enables early treatment. Biomarker measurement using a patient biological sample is more expeditious than implementing a brain scan as well as being technically more simplistic and economical. Glial fibrillary acidic protein (GFAP) is a ~50 kDa (kDa = kilodaltons) structural protein found predominantly in the brain. It is essentially undetected in healthy patient systemic serum but is released in increasing amounts during HS (EP1668370B1 - intracerebral stroke, WO2005/05029088). The utility of GFAP as a biomarker of HS is becoming increasingly recognised but there is always a need for further stroke biomarkers, especially those which provide greater sensitivity and greater accuracy in diagnosing and predicting HS and which support improvements in the differential diagnosis of stroke.

Changhong et al., (2016, Scientific Reports) relates to the assessment of serum UCH-L1 and GFAP in acute stroke patients. Wunderlich et al., (2006, European Journal of Neurology) relates to the release of GFAP being related to neurovascular status in acute ischemic stroke. Mayer et al., (2013, PLOS One) relates to blood levels of GFAP in patients with neurological disease. Chen et al., (2013, ASN Neuro) relates to caspase cleavage of GFAP producing an assembly-compromised proteolytic fragment that promotes filament aggregation. Papa et al., (2012, Annals of Emergency Medicine) relates to elevated levels of serum GFAP breakdown products in mild and moderate traumatic brain injury being associated with intracranial lesions and neurosurgical intervention. Kimikazu et al., (1998, Brain Research) relates to an increase of GFAP in the spinal cord of a motor neuron degeneration mutant mouse. Suzuki et al., (2005, Neurological Research) relates to the identification of nitrated proteins in the normal rat brain using a proteomics approach.

### SUMMARY OF INVENTION

Described are methods of supporting the diagnosis of stroke events in patients using derivatives of GFAP. It has been unexpectedly found that GFAP derivatives make up a substantial proportion of total GFAP protein in samples taken from individuals undergoing a stroke event, and their detection and measurement enables improved assays for the diagnosis and prognosis of stroke. GFAP derivatives are GFAP fragments or species, with or without post translational modification (PTM), derived from native GFAP (parent GFAP). By detecting and measuring total GFAP, GFAP derivatives plus native GFAP, a more sensitive assay results. The invention further describes antibody-based detection methods which through epitopic targeting of amino acid sequences common and/or unique to the various GFAP species, enables assays for improved stroke prediction and diagnosis. These methods can be used independently or can be combined with brain scanning in patients displaying stroke symptoms to predict, diagnose and discriminate fatal ischaemic stroke (IS fatal), haemorrhagic transformation (HT) and haemorrhagic stroke (HS), thus enabling timely and accurate diagnoses, patient treatment prioritisation and more efficient patient management. Also described are antibodies which target areas of the various species of GFAP, peptide domains of GFAP derivatives or native GFAP which incorporate the epitopes bound by the antibodies of the invention that enable the methods of the invention.

### FIGURES

Figure 1 Graphs of GFAP concentration in pernicious and non-pernicious stroke for Kit 1 and Kit 2 as described in the Methods Examples and Results section.

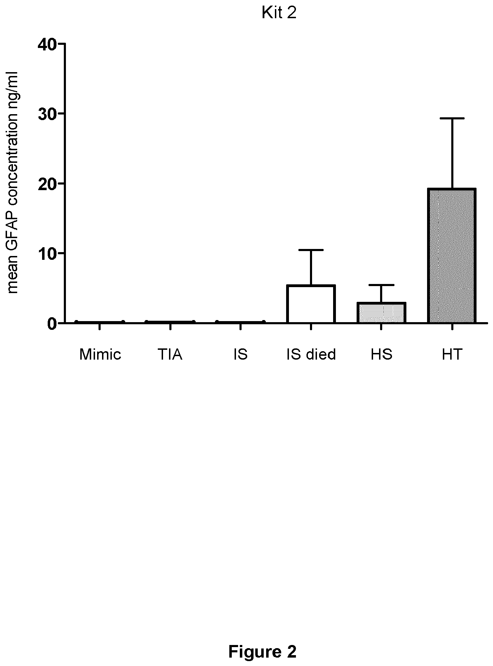

Figure 2 Graph of the mean (standard deviation in brackets) GFAP concentrations in subjects with stroke mimics, TIA, IS, IS fatal (IS died), HS and HT.

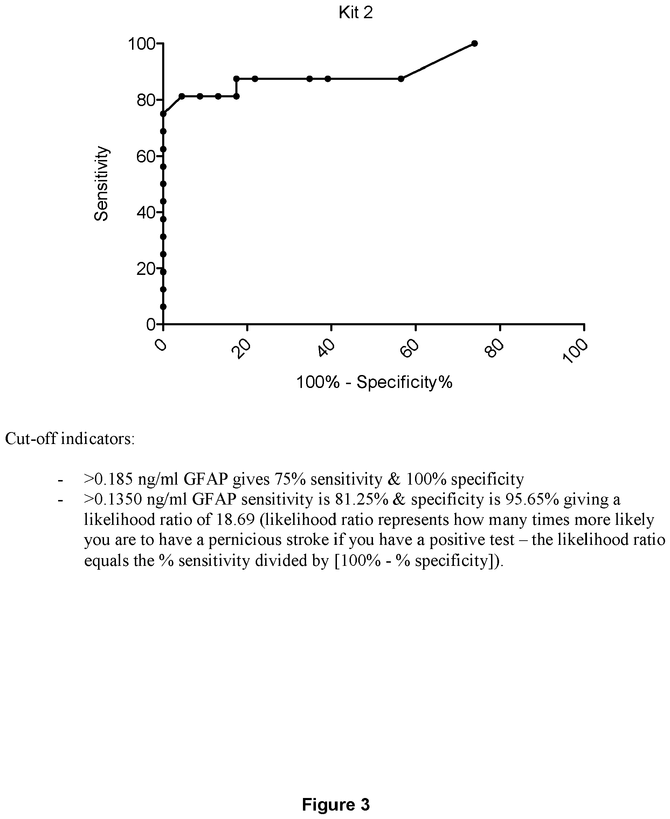

Figure 3 ROC Curve of pernicious stroke vs non-pernicious stroke using Kit 2.

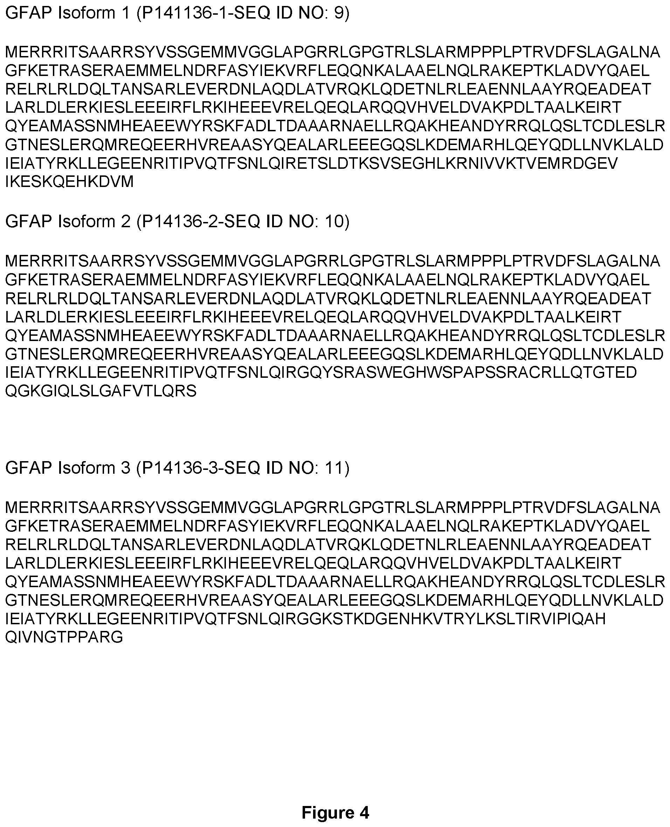

Figure 4 Sequences of the main three GFAP isoforms (Taken from the Uniprot database - www.uniprot.org, www.uniprot.org/uniprot/?query=gfap&sort=score).
Figure 5 GFAP peptide fragments, native GFAP and biochip control (FITC-fluorescine isothiocyanate) used in epitope mapping of antibodies GF9, GF10, 4A11 and GA-5 (or 'GA5'). The fragments were spotted on discrete test regions (DTR) on a biochip.
Figure 6 The biochip spotting configuration corresponding to Figure 5 used to derive the binding characteristics of antibodies GF9, GF10, 4A11 and GA-5.
Figure 7 Biochip images following antibody addition to the biochip spotting configurations described in Figures 6. Arrows indicate unique binding by antibody GF9 to DTR 4 incorporating GFAP peptide sequence ETRASERAEMME (SEQ ID NO: 1) and antibody GF10 to DTR 2 incorporating GFAP peptide sequence ERKIESLEEEIR (SEQ ID NO: 2).
Figure 8 GFAP peptide fragments, native GFAP and biochip control (FITC-fluorescine isothiocyanate) used in epitope mapping of antibodies 4A11 and GA-5. The fragments were spotted on discrete test regions (DTR) on a biochip.
Figure 9 The biochip spotting configuration corresponding to Figure 8 used to derive the binding characteristics of antibodies 4A11 and GA-5 (above). Biochip images following antibody addition to the biochip spotting configurations (below). These shows unique binding by antibody 4A11 to DTR 26 incorporating GFAP peptide sequence NLRLEAENNLAA (SEQ ID NO: 3) and antibody GA-5 to DTR 37 incorporating GFAP peptide sequence VQTFSNLQIR (SEQ ID NO: 4).

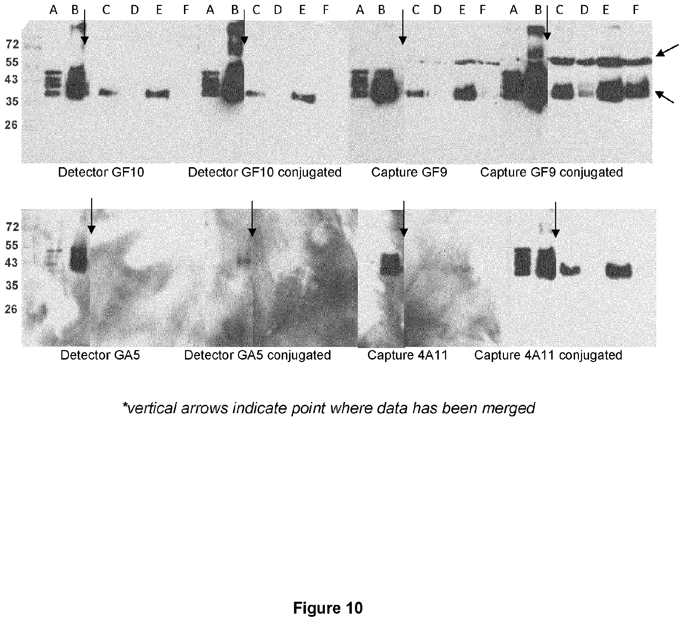

Figure 10 Western blot images (see Methods Examples and Results for experimental methodologies) using the four antibodies of Kits 1 and 2 challenged with cerebrospinal fluid samples taken from patients who have undergone subarachnoid haemorrhage. Lane far left is standard (ladder), Lane A is GFAP (Calbiochem Cat. No. 344596)), Lane B is human brain tissue lysate (ProSci), Lanes C to F represent CSF samples of subarachnoid haemorrhage patients. As can be seen from the images antibody GF9 binds to unique GFAP derivatives of molecular weight greater and less than native GFAP (indicated by arrows to the right of the upper image)
Figure 11 Western Blot images (see Methods Examples and Results for experimental methodologies) of the GF9 antibody challenged with serum samples taken from haemorrhagic stroke patients. Lane A is native GFAP (Calbiochem), Lane B is human brain tissue lysate (ProSci), Lanes C to F represent serum samples of haemorrhagic stroke patients. As can be seen from the image antibody GF9 binds to a GFAP derivative of molecular weight less than native GFAP. This GFAP derivative is also present in human brain tissue lysate.

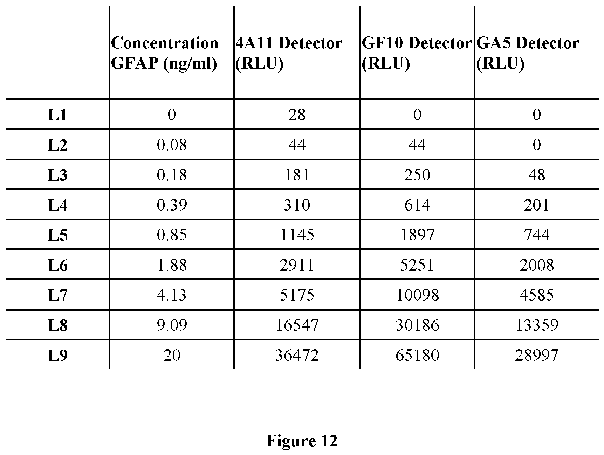

Figure 12 Relative light unit (RLU) values for generating a standard curve derived from a sandwich assay for native GFAP using GF9 capture antibody with 4A11, GF10 and GA-5 detector antibodies bound to HRP
Figure 13 Concentration of GFAP species (GFAP derivatives of MW less than native GFAP according to Western Blot) in samples derived from stroke patients. The patient samples were cerebrospinal fluid (CSF), serum and plasma. The concentration values were derived using a GF9 capture antibody and a 4A11 detector antibody bound to HRP. The sandwich assay was conducted using the Randox Laboratories Evidence Investigator^{™} system.
Figure 14 Concentration of GFAP species (GFAP derivatives of MW less than native GFAP according to Western Blot) in CSF and serum samples derived from stroke patients as derived using GF9 capture antibody and 4A11 and GA5 detector antibodies.

### DETAILED DESCRIPTION OF THE INVENTION

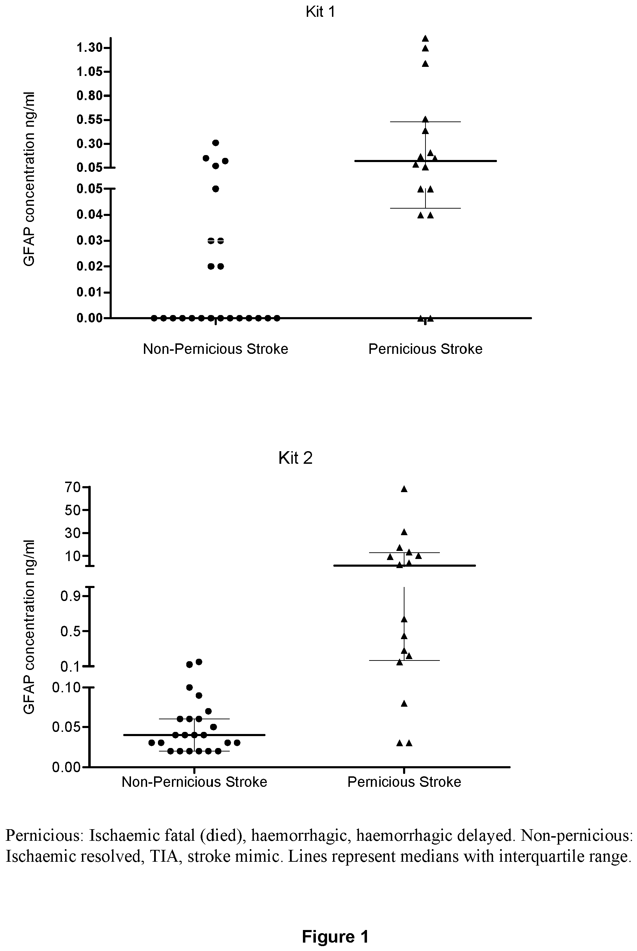

During a routine clinical study assessing the utility of native GFAP as a biomarker for haemorrhagic stroke it has been surprisingly found that GFAP derivatives of molecular weight less than native GFAP without PTM represent a major component of total GFAP present in stroke patient samples. The amount of these GFAP derivatives is higher in HS patients, HT patients and fatal IS patients compared to control values. It was also found that further GFAP derivatives, namely native GFAP with post translational modifications and/or GFAP derivatives with a molecular weight of greater than native GFAP without PTM, were also present in the cerebrospinal fluid (CSF) of stroke patients. This enables the use of GFAP derivatives in a clinical or point of care setting as biomarkers of various stroke conditions to inform and support the clinical decision-making process. These various stroke conditions include HS (intracerebral haemorrhage and/or subarachnoid haemorrhage), HT and IS fatal. The use of these GFAP species enables an assay of increased diagnostic power. The assay is more accurate as it avoids categorising samples as devoid of GFAP when GFAP is present (see Figure 1). The following definitions are used throughout this specification:
*'Native GFAP'* means, unless otherwise stated, one or more of the three characterised GFAP full-length isoforms listed in the Uniprot database, GFAP isoform 1 of Uniprot number P14136-1 synonym GFAP alpha, GFAP isoform 2 of Uniprot number P14136-2 synonym GFAP delta, GFAP isoform 3 of Uniprot number P14136-3 synonym GFAP epsilon, each with or without PTM. GFAP isoform 1 has a mass of 49,880 Da, GFAP isoform 2 has a mass of 50,289 Da, GFAP isoform 3 has a mass of 49,508 Da. Herein a compound may be referred to by i. Uniprot number ii. chemical structure iii. chemical name; in the event of conflict the order of priority for compound identification is i. > ii > iii.

*'GFAP derivative(s)'* means one or more individual GFAP species with or without PTM of molecular weight less than native GFAP without PTM i.e. one or more GFAP species of a molecular weight of less than about 49 kDa (see definition of `native GFAP' for the three reference native GFAP species and their molecular weights) e.g. a GFAP fragment derived from native GFAP that has been enzymatically digested, and/or one or more individual GFAP species with post translational modification of molecular weight greater than native GFAP without PTM i.e. of a molecular weight of greater than about 51 kDa e.g. a hyperglycosylated GFAP fragment of a molecular weight which exceeds about 51 kDa; the definition 'GFAP derivative(s)' excludes native GFAP with PTM.

*'Total GFAP'* means native GFAP and GFAP derivatives.

*'GFAP* species' means any GFAP-based protein including all native GFAP isoforms with and without PTM and other GFAP isoforms such as GFAP beta, GFAP kappa, GFAP gamma, GFAPΔEx6, GFAPΔ135, GFAPΔ164 and GFAPΔEx7 (see Moeton et al 2016), GFAP multimers (dimers, tetramers etc based on any of the various isoforms) and their fragments with or without PTM, and GFAP derivatives with or without PTM.

'IS fatal' means individuals diagnosed with ischaemic stroke who subsequently die.

'Pernicious stroke' means a type of stroke in which the individual has been subject to or is undergoing a potentially life-threatening stroke event. Pernicious stroke can be HS, HT or IS fatal.

`Haemorrhagic stroke' includes both intracerebral stroke and subarachnoid stroke unless otherwise stated.

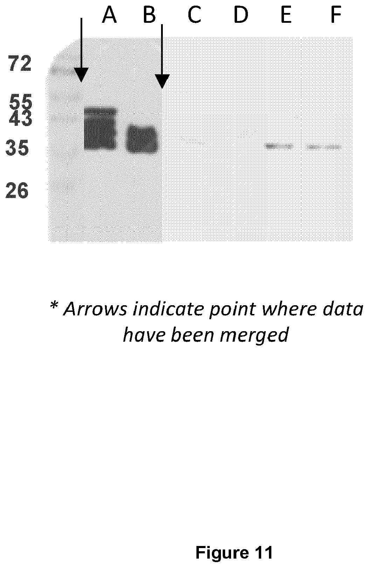

The invention describes a method of diagnosing, or supporting the diagnosis of,stroke in a subject comprising measuring the amount of GFAP derivative comprising the sequence ETRASERAEMME (SEQ ID NO: 1) present in a sample which has been taken from the subject, the method incorporating an antibody which binds to an epitope of the GFAP derivative within the sequence ETRASERAEMME (SEQ ID NO: 1), wherein an amount of GFAP derivative in the sample bound by the antibody which is greater than the amount of the GFAP in a control subject is indicative of stroke. The GFAP derivatives may be of molecular weight less than native GFAP without PTM. Reference to GFAP derivatives includes instances where there exist in a sample i) only GFAP derivatives with PTM ii) only GFAP derivatives without PTM and iii) a mixture of GFAP derivatives, some with and some without PTM. In a preferred embodiment, the GFAP derivative or derivatives of molecular weight less than native GFAP without PTM has or have a molecular weight of about 35 - 49 kDa, 35 - 42 kDa, - 49 kDa or 35 - 39 kDa;. Preferably, the identified stroke is one or more of haemorrhagic stroke, haemorrhagic transformation and fatal ischaemic stroke. In a preferred embodiment the method comprises: ≤ 24 hours from admission or symptom onset measuring the amount of GFAP derivatives, in which GFAP derivatives are of molecular weight less than native GFAP without PTM, in a biological sample taken from the patient and subjecting the patient to a brain scan and, if the GFAP derivatives value is normal and the brain scan negative for haemorrhagic stroke, at ≥ 24 hours from admission or symptom onset taking a further sample from the patient and measuring the amount of the GFAP derivatives, in which i) a high GFAP derivatives protein level and a brain scan positive for haemorrhagic stroke ≤ 24 hours from admission or symptom onset indicates haemorrhagic stroke ii) a high GFAP derivatives protein level and a brain scan negative for haemorrhagic stroke ≤ 24 hours from admission or symptom onset indicates fatal ischaemic stroke iii) a normal GFAP derivatives protein level and brain scan negative for haemorrhagic stroke on admission and a high total GFAP or GFAP derivatives protein level ≥ 24 hours from admission or symptom onset indicates haemorrhagic transformation. Preferably, samples are taken from a subject ≤ 6 hours from subject admission or symptom onset. Preferably, the second glial fibrillary acidic protein measurement is taken at 24 - 96 hours from admission or from symptom onset. Other possible time points for taking a subject sample is at ≤ 12 hours, ≤ 8 hours or ≤ 4 hours from subject admission or symptom onset. Reference to an 'amount' of one or more GFAP species is replaceable by other suitable quantifying terms such as 'concentration', 'level' etc. In all of the methods of the invention it is preferable that the time of symptom onset is the defining reference point. However, circumstances may not allow this due to the subject's condition or lack of third party verification, in which case admission time (which can also include medic arrival at the scene) is used. The sample to be analysed in the methods of the invention can be any suitable biological sample such as blood, serum, plasma, urine, tissue, saliva; the biological sample is preferably a blood sample, a serum sample or a plasma sample. Native GFAP can also be measured in conjunction with GFAP derivatives. In variations of the described method of the invention and in further embodiments of the described method of the invention of diagnosing or supporting the diagnosis of stroke in a subject i) native GFAP and GFAP derivatives of molecular weight less than native GFAP without PTM present in the sample are measured, in which an amount measured which is greater than the amount of a GFAP control is indicative of stroke ii) GFAP derivatives of molecular weight less than native GFAP without PTM and GFAP derivatives of molecular weight greater than native GFAP without PTM present in the sample are measured, in which an amount measured which is greater than the amount of a GFAP control is indicative of stroke iii) GFAP derivatives of molecular weight greater than native GFAP without PTM present in the sample are measured, in which an amount measured which is greater than the amount of a GFAP control is indicative of stroke iv) native GFAP and GFAP derivatives of molecular weight greater than native GFAP without PTM present in the sample are measured, in which an amount measured which is greater than the amount of a GFAP control is indicative of stroke v) native GFAP and all GFAP derivatives present in the sample are measured (i.e. total GFAP) in which an amount measured which is greater than the amount of a GFAP control is indicative of stroke. Preferably, the identified stroke is one or more of haemorrhagic stroke, haemorrhagic transformation and fatal ischaemic stroke. An immuno-based (i.e. antibody-based) technique is the preferred analytical technique, and can be, for example a biochip array technology (BAT) based sandwich or competitive immunoassay format or a lateral flow point of care (POC) format. For the preferred sandwich immunoassay format, two or more antibodies of differing specificity can be used as capture antibodies for the native GFAP and the GFAP derivatives of molecular weight less or more than native GFAP without PTM, together with either a single detector antibody detecting a single epitope common to total GFAP species, or with two or more detector antibodies of differing epitope specificity. However, in a preferred format, a single capture antibody specific to an epitope common to the various GFAP species, and a single detector antibody specific to a different epitope common to the various GFAP species, are used e.g. the antibodies of Kit 2. Preferably, the method comprises: ≤ 24 hours from admission or symptom onset measuring the amount of total GFAP in a biological sample taken from the patient and subjecting the patient to a brain scan and, if the total GFAP value is normal and the brain scan negative for haemorrhagic stroke, at ≥ 24 hours from admission or symptom onset taking a further sample from the patient and measuring the amount of total GFAP in which i) a high total GFAP protein level and a brain scan positive for haemorrhagic stroke ≤ 24 hours from admission or symptom onset indicates haemorrhagic stroke ii) a high total GFAP protein level and a brain scan negative for haemorrhagic stroke ≤ 24 hours from admission or symptom onset indicates fatal ischaemic stroke iii) a normal total GFAP protein level and brain scan negative for haemorrhagic stroke on admission and a high total GFAP protein level ≥ 24 hours from admission or symptom onset indicates haemorrhagic transformation. Preferably, samples are taken from a subject ≤ 6 hours from subject admission or symptom onset. Other possible time points for taking a subject sample is at ≤ 12 hours, ≤ 8 hours or ≤ 4 hours from subject admission or symptom onset. Preferably, the second glial fibrillary acidic protein measurement is taken at 24 - 96 hours from admission or from symptom onset. In each of the previous methods a measured GFAP concentration greater than a GFAP control value is indicative of haemorrhagic stroke, haemorrhagic transformation or fatal ischaemic stroke; preferably, the level of GFAP in the stroke condition is ≥ 1.1 times, ≥ 1.2 times, ≥ 1.5 times ≥ 2 times, ≥ 3 times, or ≥ 4 times than the control value. These values reflect the fact that GFAP is at very low or undetectable levels in control ('normal') samples.Thus the methods of the invention incorporating measurement of GFAP derivatives, can be used in the diagnosis of HS, HT and IS fatal. The methods of the invention can incorporate any suitable antibodies but it is preferable that an antibody which binds to an epitope of a GFAP species within the sequence ETRASERAEMME (SEQ ID NO: 1) is incorporated in the methods. Preferably, the epitope comprises a least one amino acid from the sequence SERA (SEQ ID NO: 5) within ETRASERAEMME (SEQ ID NO: 1). When the immunoanalytical method is a standard sandwich immunoassay, the ETRASERAEMME (SEQ ID NO: 1) binding antibody can be the detector or capture antibody. It is preferably the capture antibody. The capture antibody of the immunoassay can be in solution, in solution bound to a solid-state device such as a bead, microparticle or magnetic particle or adsorbed or chemically attached to a substrate such as a biochip. A biochip is a planar substrate that may be, for example, mineral or polymer based, e.g. glass or plastic, but is preferably ceramic. The solid-state devices used are optionally adapted by being partially or completed covered by a material which promotes an improved assay e.g. a hydrophobic composition. The preferred substrate is a ceramic biochip optionally completely or partially covered with a hydrophobic composition. Hydrophobic compositions, well known in the art, can help minimise or prevent non-specific binding, optimise antibody-containing droplet characteristics when added to the solid-state device, mitigate signal bleeding thus improving detection and quantification. The immunoanalytical method can incorporate a further antibody. For example, the preferred sandwich format includes a further antibody which binds to an epitope which is either common to GFAP derivatives or common to total GFAP species, the epitope bound by the further antibody differing from the epitope bound by the first antibody. The epitope specificity of each of the two antibodies of the immunoassay are sufficiently different that one antibody does not impede or interfere with the binding of the other antibody to its epitope and vice-versa, and that the integrity of the immunoassay in detecting and quantifying GFAP derivatives and/or total GFAP is not compromised. It is preferred that for all immunoanalytical methods used for the diagnosis of stroke that the further antibody binds to an epitope of a GFAP species incorporated in the sequence ERKIESLEEEIR (SEQ ID NO: 2). The GFAP control level as used in the described methods of the invention is one that has been measured in a healthy patient or a healthy population of patients, or has been derived from a population of patients categorised as one or more of TIA, stroke mimic or ischaemic stroke. A 'control', unless otherwise qualified, can be native GFAP, a GFAP derivative or any other GFAP species including other GFAP isoforms and their derivatives and GFAP dimers and oligomers and their derivatives, or a mixture of two or more GFAP species that has been measured in a healthy patient or a healthy population of patients or has been derived from a population of patients categorised as one or more of TIA, stroke mimic or ischaemic stroke (the control population); ischaemic stroke patient data can be used as a control if, as shown by current study, GFAP levels do not increase in such patients. The invention further describes the use of GFAP derivatives comprising the sequence ETRASERAEMME (SEQ ID NO: 1) as markers of stroke, wherein the sequence ETRASERAEMME (SEQ ID NO: 1) is the target of an antibody in a diagnostic assay. The GFAP derivatives can be GFAP derivatives of molecular weight less than native GFAP without PTM and GFAP derivatives of molecular weight more than native GFAP without PTM (i.e. GFAP fragments with substantial PTM). Preferably GFAP derivatives of molecular weight less than native GFAP with or without PTM are used. The GFAP derivatives are used to support the diagnosis or diagnose haemorrhagic stroke, haemorrhagic transformation and fatal ischaemic stroke. In a further embodiment, the use is effected in conjunction with a brain scan. Brain scan includes computerised tomography (CAT scan), X-ray, magnetic resonance imaging (MRI), ultrasound or any other machine-based (medical device) scan in which the interaction of the brain with wave or particle output from a medical device enables an abnormal brain event to be highlighted, an abnormal brain event being one which potentially injurious to the brain. The uses described are applicable to GFAP derivatives incorporating the sequence ETRASERAEMME (SEQ ID NO: 1). The invention also describes an antibody which binds to an epitope of a GFAP species incorporated in the sequence ETRASERAEMME (SEQ ID NO: 1). The epitope bound by the antibody can be two, three, four, five, six, seven or more contiguous amino acid residues. Experimental data show that the two peptide sequences which overlap the sequence ETRASERAEMME (SEQ ID NO: 1), namely sequences GALNAGFKETRA (SEQ ID NO: 6) and EMMELNDRFASY (SEQ ID NO: 7), do not bind to the antibody which suggests that one or more of the central, non-overlapping residues of ETRASERAEMME (SEQ ID NO: 1), namely SERA (SEQ ID NO: 5), likely comprise the epitope. Hence in a further embodiment, the invention describes an antibody in which the epitope comprises a least one amino acid from the sequence SERA (SEQ ID NO: 5) of ETRASERAEMME (SEQ ID NO: 1). The GFAP species bound by the antibody is preferably a GFAP derivative of molecular weight less than native GFAP with or without PTM and/or native GFAP with PTM. The antibody can bind a GFAP derivative corresponding to the lowest molecular weight GFAP species in the Calbiochem standard which implies that the GF9 and GF10 antibodies bind to a GFAP derivative of molecular weight of about 44 kDa.(see Figures 10 & 11 and Calbiochem datasheet which describes the GFAP standard as having a molecular weight of 43 to 49 kDa); the GF9 antibody can bind to other GFAP derivatives of molecular weight less or greater than about 43 kDa but less than about 49 KDa as well as GFAP species of molecular weight greater than about 49 kDa (see upper two gels furthest to right of Figure 10). The utility of the antibody and methods of the invention will be recognised for other GFAP species, including the GFAP isoforms GFAP beta, GFAP kappa, GFAP gamma, GFAPΔEx6, GFAPΔ135, GFAPΔ164 and GFAPΔEx7 (Moeton et al 2016). The antibody can be attached (chemically bonded e.g. by covalent bonding) to or adsorbed on the surface of a solid-state device such as a bead, microparticle, microtitre plate or biochip. To be chemically attached the surface of the solid-state device is usually chemically activated or modified in order to render the surface amenable to antibody attachment. Likewise, the antibody may also be chemically activated or modified to make it amenable to attachment to the solid-state device. The antibody of the invention in all its embodiments is used in the diagnosis of stroke, stroke preferably being HS, HT or fatal IS. The antibody of the invention, in all its embodiments, can be incorporated into a kit. The invention further describes an antibody which binds to an epitope of a GFAP species incorporated in the sequence ERKIESLEEEIR (SEQ ID NO: 2); this antibody can be either the detector or capture antibody and can be attached (chemically bonded e.g. by covalent bonding) to or adsorbed on the surface of a solid-state device such as a bead, microtitre plate or biochip. To be chemically attached the surface of the solid-state device is usually chemically activated or modified in order to render the surface amenable to antibody attachment. Likewise, the antibody may also be chemically activated or modified to make it amenable to attachment to the solid-state device. The aforementioned kit can also incorporate an antibody binding to an epitope of a GFAP species incorporated in the sequence ERKIESLEEEIR (SEQ ID NO: 2). The invention further describes a capture antibody and detector antibody each binding a discrete epitope of non-overlapping amino acid residues comprised within the sequence 60 to 383 of GFAP corresponding to GFAP uniprot numbers P14136-1, P14136-2, and P14136-3 (SEQ ID NO: 9, SEQ ID NO: 10 and SEQ ID NO: 11) for use in diagnosing or supporting the diagnosis of stroke; the antibodies can be GF9 and GF10 as well as the commercially available antibodies 4A11, SMI-21, SMI-23, SMI-24, SMI-25, 6F2 and 2.2B10 (see Lin 2017, PloS One, Characterisation of a panel of monoclonal antibodies recognising specific epitopes on GFAP) in capture-detector combinations in which the GFAP species epitope bound by the capture antibody is discrete from and does not interfere with (i.e. does not undermine the binding of) the epitope bound by the detector antibody. Preferred capture-detector antibody combinations for use in the methods of the invention are GF9 and GF10, and GF9 and 4A11, in which for both pairs each of the two antibodies within a pair can be the capture antibody or the detector antibody, but it is preferable that GF9 is the capture antibody for each pair. A preferred antibody capture-detector combination for use in an immunoassay method or kit for binding GFAP species is 4A11 and GF9, especially when the GF9 antibody is the capture antibody. By targeting residues 60 to 383 common to the GFAP isoforms corresponding to Uniprot numbers P14136-1, P14136-2, and P14136-3 (SEQ ID NO: 9, SEQ ID NO: 10 and SEQ ID NO: 11), using a sandwich immunoassay format, an assay of improved sensitivity can be achieved. Residues 60 and 383 correspond to terminal amino acids following native GFAP exposure to the enzyme calpain. Scission of one or both of these residues within native GFAP gives rise to the following GFAP derivatives in which the given number range corresponds to the amino acid residue range of SEQ ID NO: 9 (GFAP Uniprot number P14136-1) and MW is the approximate molecular weight (brackets is the exact molecular weight based on GFAP without PTM): GFAP 1-59 MW= ~ 6,000 Da (6,339 Da), GFAP 384-432 MW= ~ 6,000 Da (5,670 Da), GFAP 60-383 MW= ∼ 38,000 Da (37,906 Da), GFAP 1-383 MW= -44,000 Da (44,228 Da), GFAP 60-432 MW= ∼ 44,000 Da (43,559 Da). A comparable approach can be used with GFAP Uniprot numbers P14136-2 and P14136-3 (SEQ ID NO: 10 and SEQ ID NO: 11). One or more of these GFAP derivatives could be used as biomarkers of stroke or support the diagnosis of stroke and be used in the methods and products of the invention described herein.

The invention describes the use of GFAP derivatives as markers of stroke, especially haemorrhagic stroke, haemorrhagic transformation or fatal ischaemic stroke; the GFAP derivative can be of any molecular weight less than native GFAP without PTM and can be of about 35 kDa, 36 kDa, 37 kDa, 38 kDa, 39 kDa, 40 kDa, 41 kDa, 42 kDa, 43 kDa, 44 kDa, 45 kDa, 46 kDa, 47 kDa, 48 kDa and 49 kDa. Protein gels are a standard technique used in protein separation and assignment of approximate molecular weights and the term 'about' reflects this. The Calbiochem product number 344596 is a GFAP standard purified from human brain having a molecular weight between 43 and 49 kDa (datasheet). Reference to the Calbiochem standard (Lane A) of the gels of Figures 10 and 11 and the epitopic binding characteristics of antibody GF9 implies a GFAP derivative of MW= 43,000 - 44,000 Da being increased in the samples of stroke patients. This could correspond to either the amino acid sequence 60-432 or 1-383 of GFAP Uniprot number P14136-1 (SEQ ID NO: 9) generated by calpain digestion; given the proximity of the GF9 epitope to the calpain 59-60 digest point, GFAP derivative 60 - 432 is a possibility. The gels of Figure 10 highlight other GFAP derivatives of MW= 43-49 kDa and of MW > 49 kDa at about 55 kDA that are produced in samples of stroke patients and that can also be used in diagnosing or supporting the diagnosis of stroke.

Preferably the GFAP derivative or derivatives bind to an antibody, the antibody binding an epitope within the sequence ETRASERAEMME (SEQ ID NO: 1) of the GFAP derivative(s), preferably to at least one amino acid within the sequence SERA (SEQ ID NO: 5) of the GFAP derivative. The antibody is preferably GF9. As used herein, the term `ischaemic stroke (IS)' refers to the type of stroke that occurs when blood supply to the brain is decreased, resulting in brain damage. An ischaemic stroke occurs when a blood vessel becomes blocked, usually via a blood clot. This clot may form locally at an atherosclerotic plaque (thrombotic stroke) or alternatively may occur due to a travelling particle or debris that has originated from elsewhere in the bloodstream (embolic stroke). The term 'transient ischaemic attack (TIA)' refers to a 'mini stroke' that occurs when blood supply to the brain is temporarily decreased. A TIA is diagnosed if symptoms are quickly resolved (within 24 hours with the individual returning to normal health). The term `stroke mimic' applies to non-stroke conditions that produce one or more symptoms associated with stroke and which leads to the individual's condition initially categorised as possible stroke. The term 'haemorrhagic stroke (HS)' occurs when blood accumulates within the skull vault, usually when a weakened blood vessel ruptures. Haemorrhagic stroke can be classified into two major sub-types: intracerebral (within the brain tissue); and subarachnoid (around the surface of the brain and under its protective layer). Haemorrhagic transformation (HT) or a haemorrhagic transformer describes an individual initially diagnosed as ischaemic stroke who at a later point develops haemorrhagic stroke (see Jickling 2014 for a review of HT). References herein to 'a patient who has suffered or is suffering a stroke' include a patient who has been diagnosed as currently suffering from a stroke or who is or has been diagnosed as having previously had a stroke. The stroke may have been a recent event, such an event having initiated the process of the individual seeking clinical help. The terms "subject" and "patient" may be used interchangeably herein and refer to a mammal including a non-primate (e.g. a cow, pig, horse, dog, cat, rat and mouse) and a primate (e.g. a monkey and human). Preferably the subject or patient is a human. As used herein, the term 'biomarker' refers to a molecule present in a biological sample obtained from a patient, the concentration of which in said sample may be indicative of a pathological state. GFAP derivatives and total GFAP may be used as complementary biomarkers to other stroke biomarkers to increase the diagnostic power of a stroke assay. As used herein, the term 'complementary biomarker' refers to a biomarker that can be used in conjunction with other stroke biomarkers to support diagnosis; thus GFAP derivatives and GFAP parent with one or more stroke biomarkers are contemplated for use in stroke diagnosis. It is well understood in the art that biomarker normal or 'background' concentrations may exhibit slight variation due to, for example, age, gender or ethnic/geographical genotypes. As a result, the cut-off value used in the methods of the invention may also slightly vary due to optimization depending upon the target patient/population. The biological sample obtained from a patient is preferably a blood, serum or plasma sample. As used herein, the term *'ex vivo'* has its usual meaning in the art and refers to a sample that has been removed from a patient's body. When a blood sample is taken from the patient for analysis, whole blood, serum or plasma is analysed. Analysis of the blood sample can be by way of several analytical methodologies such as mass spectrometry linked to a pre-separation step such as chromatography. The preferred methodology is based on immuno-detection. Immuno-detection technology is also readily incorporated into transportable or hand-held devices for use outside of the clinical environment. A quantitative immunoassay such as a Western blot or ELISA can be used to detect the amount of protein. A preferred method of analysis comprises using a multi-analyte biochip which enables several proteins to be detected and quantified simultaneously. 2D gel electrophoresis is also a technique that can be used for multi-analyte analysis. Although polyclonal or monoclonal antibodies are the preferred binding agents used in the invention, short chain antibody fragments, single chain fragments and other antibody-based probes may be used. Preferably, the solid-state device used in the methods of the present invention is a microtitre plate, preferably the Biochip Array Technology system (BAT) (available from Randox Laboratories Limited). More preferably, the Evidence Evolution, Evidence Investigator and Evidence Multistat apparatus (available from Randox Laboratories) may be used to determine the levels of biomarkers in the sample.

### Glial Fibrillary Acidic Protein.

GFAP is a class-III intermediate filament (IF) protein localised to CNS astrocyte cells, gastrointestinal Schwann cells, enteric glial cells and retinal Muller cells. Approximately ten GFAP isoforms have been identified however most clinical research remains focused on isoform 1 which is the predominant GFAP expressed in CNS astrocyte cells. It is increasingly evident that GFAP is subject to complex patterns of PTM which defines its physiological function and role in pathological progression. A pivotal physiological parameter, phosphorylation, constitutes a key PTM event that defines transition between phosphorylated monomeric and dimeric unphosphorylated GFAPs that integrate with other proteins to generate a stable cellular cytoskeletal structure. In contrast, GFAP hyper-phosphorylation has been implicated in disease progression driving a loss of cellular integrity. A defining parameter of acute brain pathophysiology results in increased intracellular calcium sequestration and a shift in ionic balance which is excitotoxic, activating a number of damaging intracellular cascades, including lipid peroxidation and free-radical generation. Since citrullination or deamination is a calcium-dependent transformation of arginine residues performed by members of the peptidyl-arginine deiminases family it has been proposed to play a causative role in multiple neurodegenerative disorders. Additionally, protein citrullination is well documented as a defining parameter in autoimmune disease and GFAP autoantibodies are characteristic in the systemic circulation of stroke and TBI patients. Citrullinated GFAP has also been observed in neurodegenerative pathologies such as Alzheimer's brain tissue, while a complex profile of citrullinated GFAP has been detected in the brains of multiple sclerosis patients. Increased citrullination has also been detected in the retinae and optic cups of age-related macular degeneration and in glaucomatous optic nerves. Protein carbonylation is a modification indicative of oxidative stress that is characterised by the addition of carbonyl groups into protein-bound amino acids. Protein carbonylation can occur by direct oxidation of amino acid targets by reactive oxygen species or via interaction with reactive carbonyl species which are the product of lipid peroxidation. By either mechanism, the resulting carbonyl modifications can disrupt protein function and contribute to pathology. As a consequence, carbonylation is a metric of oxidative stress that is characteristic of various neurodegenerative pathologies, including multiple sclerosis and Alzheimer's disease. GFAP has been identified as a protein target subject to selective carbonylation. Glycosylation is one of the most common and a complex PTM events. Analysis of Alzheimer's disease tissues identified 46 soluble GFAP species. A 60% increase in the amount of more acidic GFAP species has been observed in AD the variants being both phosphorylated and N-glycosylated; more basic species were O-glycosylated and exhibited no quantitative differences between post-mortem AD and control brains. This data provides clinical evidence that the characterisation of species-specific levels of proteins in pathophysiological conditions can improve clinical diagnostics.

*Analytical Format for GFAP detection*/*quantification.* Each of the described methods of the invention can be effected using any suitable analytical technique but GFAP species are especially amenable to detection and quantification using mass spectrometry (preferably with in conjunction with a suitable chromatographic or gel based separation method, or the like) and immuno-based based methodologies; immunoanalytical methods are particularly preferred. The methods, as previously stated, preferably are based on the sandwich immunoassay format which involves a capture antibody and a detector antibody, each antibody binding to a different epitope on the target GFAP species. The capture antibody is preferably located at or chemically attached to the surface of a solid-state device. Typically, a sandwich immunoassay proceeds by way of firstly addition of the sample to be analysed, followed by addition of the detector antibody following capture antibody-analyte (GFAP species) binding. The detector antibody is conjugated to a detectable label which promotes or provides a detectable and measurable signal enabling the analyte to be detected or quantified. The signal can be any suitable electromagnetic radiation based on for example phosphorescence, fluorescence, chemiluminescence etc. Preferably, fluorescence or chemiluminescence is used. A particularly preferred detectable label is horseradish peroxidase. In a preferred immunoassay format a capture antibody of the invention is adsorbed or chemically attached to a solid state device, and after sample addition a detector antibody is added which binds to the captured GFAP species.

*Statistical Analysis.* Cut-off concentrations or values are usually derived using standard statistical techniques. A common method of biomarker statistical analysis is to use univariate methods to compare biomarker levels in various groups and highlight those biomarkers whose concentrations significantly differ across and between particular groups. This is followed by Receiver Operator Characteristic (ROC) analysis. The ROC curve is a preferred method of assessing a diagnostic test's accuracy; it addresses both the sensitivity, the number of true positives, and the specificity, the number of false positives, of the test. If two or more biomarkers are to be used in the IS diagnostic method i.e. GFAP and one or more biomarkers including DNA, RNA and protein based markers, a suitable mathematical model, such as logistic regression equation, can be derived. The logistic regression equation might include other variables such as age and gender of patient. The ROC curve can be used to assess the accuracy of the logistic regression model. The logistic regression equation can be used independently or in an algorithm to aid clinical decision making. The skilled person will be aware of numerous suitable methods for developing statistical algorithms, and all of these are within the scope of the present invention. Examples of suitable classification algorithms include multinominal logistic regression, multilayer perceptron neural network (MLP), artificial neural networks, support vector machines and random forest classifiers. To acquire a defined sensitivity and specificity the two conditions non-pernicious stroke and pernicious stroke must be demarcated using a suitable metric and this requires a reference value to be acquired for the 'normal condition' (derived from the control population), in this case the non-pernicious cohort. The normal condition could also be a healthy cohort. The reference value could be a pre-defined cut-off value which achieves the optimal sensitivity and specificity values for the target condition, a median value, mean value, quartile value etc. The methodology and analyzer used to measure a biomarker in a biological sample inevitably results in the concentration value of a particular biomarker varying. Thus, although the cut-off value, mean value etc used to determine risk of a non-pernicious or pernicious condition in a suspected stroke patient will probably vary slightly from platform to platform e.g. for an immunoassay slight variation due to, for example, the antibody (monoclonal, polyclonal etc.) or substrate (bead, elisa plate, biochip etc.), the approach using the concentration level of GFAP as a biomarker for this purpose is valid and independent of the platform and methodology used in the analytical process.

The current findings also enable a method of predicting pernicious stroke (Figure 1) in a suspected stroke patient through the measurement of GFAP derivatives and/or native GFAP utilizing the methods and antibodies described previously and in the claims.

### Methods, Examples and Results

*Patient Group.* The study consisted of 39 patients displaying stroke symptoms admitted to the Emergency Department of KAT General Hospital, Athens, Greece. Patients were classified on admission using clinical analysis incorporating the Scandinavian stroke scale and by CAT scan. Blood samples were taken at the time of admission, at 24 hrs following admission, and every 24 hours thereafter up to day six except for patients who were diagnosed as TIA and stroke mimics who were subject to a single blood sample draw on admission. The time from the onset of stroke symptoms and hospital admission was < 6 hours. Exclusion criteria included patients who were admitted to hospital > 6 hours from the time of onset of stroke symptoms, patients with hepatic or renal pathologies and patients who had previously experienced a stroke. Samples from the CSF of individuals who had undergone sub arachnoid haemorrhage were also obtained and analysed using 1D gel electrophoresis and Western Blot.

*Sample Analysis.* EDTA plasma samples of blood obtained from the patients of the study group was tested for GFAP. The proteins were detected and quantified using biochips incorporating GFAP-specific antibodies (4A11 capture antibody and GA-5 detector antibody - Kit 1) and the Evidence Investigator (Randox Laboratories Ltd, Crumlin, UK). Kit 2 comprised monoclonal antibodies (properties described in the Description) spotted on a ceramic biochip (the ceramic biochip as used for Kit 1, and as described in EP0874242). Samples from the CSF of individuals who had undergone sub arachnoid haemorrhage were analysed using 1D gel electrophoresis and Western Blot.

### SDS polyacrylamide gel electrophoresis and Western Blot.

Standard conditions were generally applied. Polyacrylamide gel (14%) was used. Samples were vortexed and incubated in a dry bath for 10-15 mins at 95°C for approximately 10-15 minutes, followed by further vortexing. Samples and MW ladder (Thermo Scientific cat. 26616) were added to appropriate lanes and the gel run at approximately 120-200V until the dye front reached the top of the gel. The Western sandwich cassette was made up of white-sponge-filter-membrane-gel-filter-sponge black. The Western was run at approximately 120V for one hour with ice pack inside mini protean and left on a stir. The blot was then blocked in 5% milk powder (20ml) for at least 30 minutes. Antibodies were added to the appropriate blots and washed with TBST (x3) for 10 minutes. If required anti-species HRP was added to the blots followed by further TBST (x4) washing. The membrane was developed using X-ray film, BM chemiluminescence Western blotting kit, developer and fixer. The use of gel data (see Figures 10 & 11), supports the characterisation of the various GFAP species present in patient samples.

*Antibody Production.* Standard techniques were implemented for monoclonal antibody production for Kit 2. Sheep were immunized with Glial Fibrillary Acidic Protein from human Brain. Lymphocytes were collected and fused with heteromyeloma cells. Supernatants from the resulting hybridoma were screened for the presence of specific antibody using ELISA based assays. Positive hybridoma were cloned to produce stable monoclonal hybridoma. The antibodies were purified and evaluated by direct binding ELISA to determine their specificity for GFAP from human brain. Further molecular work was carried out using standard antibody characterisation ELISA and SDS and Western techniques. Two antibodies were identified for further testing, antibodies GF9 and GF10 (Randox Laboratories, Crumlin, Northern Ireland).

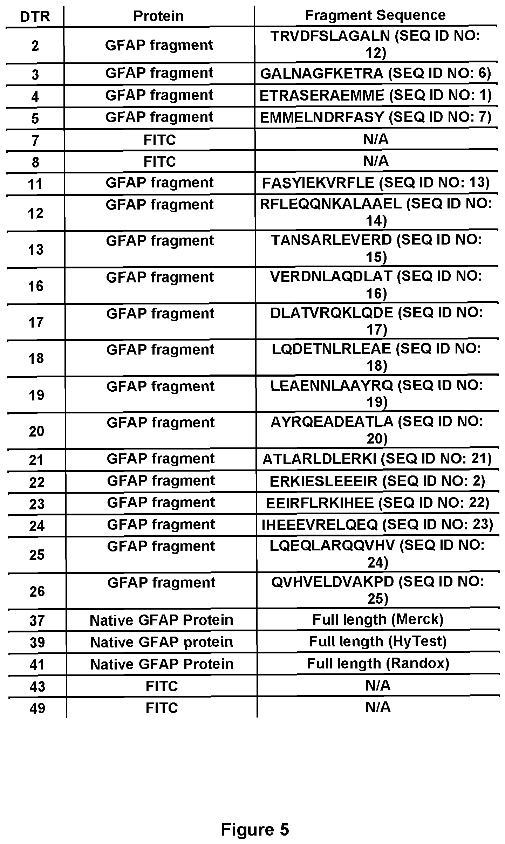

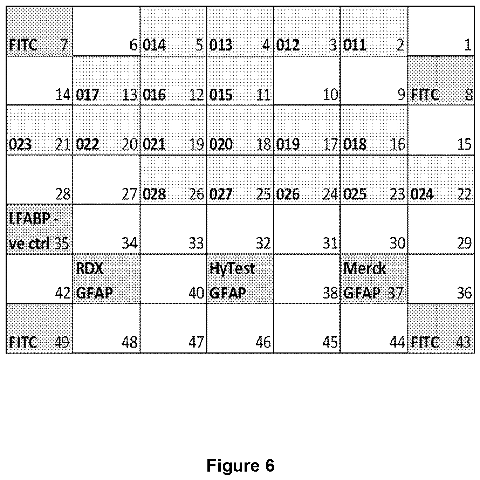

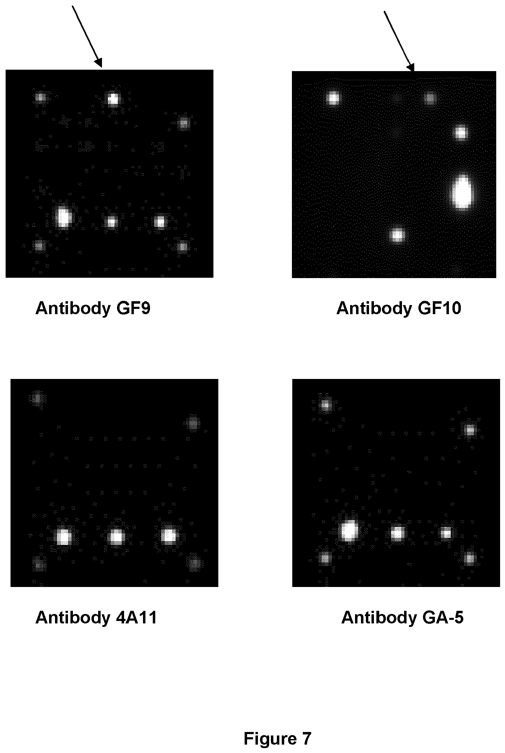

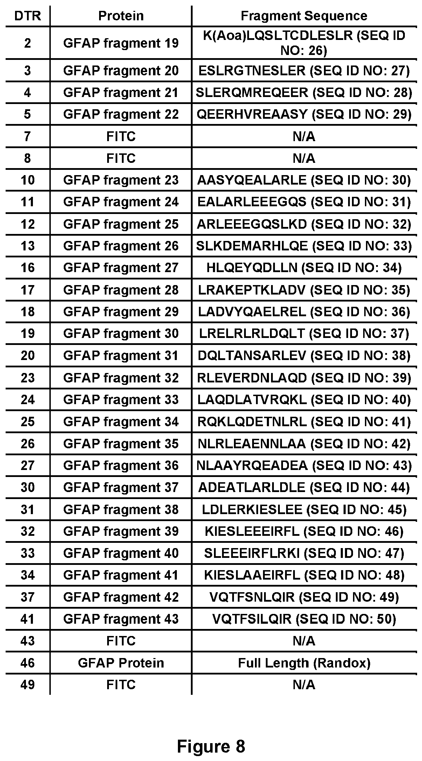

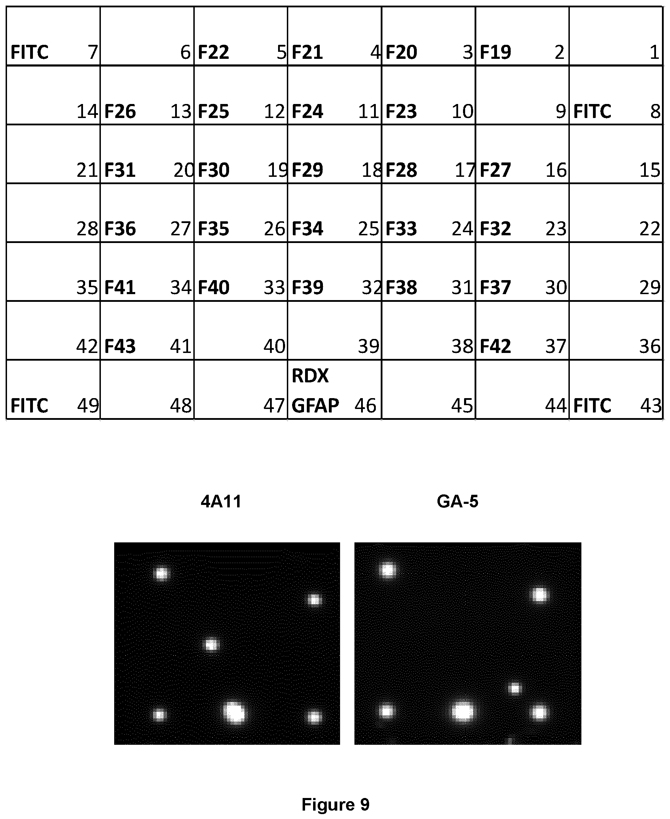

*Antibody Characterisation.* Antibody binding characterisation was effected. BSA conjugated peptides spanning amino acid residues of GFAP were generated. Individual BSA conjugated peptides were immobilised at discrete test regions (DTRs) on a Biochip, as well as control substances. The two capture and two detector antibodies from Kits 1 & 2 were conjugated to horseradish peroxidase (HRP) and each individual conjugated antibody was incubated on an individual biochip and images obtained. The test substance fragments were spotted as outlined in Figures 5 and 8 and their position on the biochip (7 x 7 grid) is shown in Figures 6 and 9 (above). The images in Figures 7 and 9 (bottom) are the four screened antibodies, the DTRs exhibiting a grey/white image confirming binding of the antibody to a spotted GFAP species or control substance e.g. all four antibodies bind to native GFAP spotted at positions 37 (Merck), 39 (Hytest) & 41 (Randox).

**Table 1 Antibodies used in sample analyses and characterisation studies**

| Clone Name | Kit |
|---|---|
| Anti-GFAP Capture GF9 | 2 |
| Anti-GFAP Detector GF10 | 2 |
| Anti-GFAP Capture 4A11 | 1 |
| Anti-GFAP Detector GA-5 | 1 |

### Results

Pernicious and non-pernicious stroke cohorts were compared using ROC curve analysis. The results are shown in Table 2 and Figure 1.

**Table 2 Statistical measures of GFAP levels using two different immunoassay kits**

| Condition | Sample number | Concentration (ng/ml) <24 hr | | P-value | AUC |
|---|---|---|---|---|---|
| *Kit 1* | | Median | Average | | |
| Non-pernicious stroke | N=23 | 0.000 | 0.034 | | |
| Pernicious stroke | N=16 | 0.120 | 0.356 | 0.0004 | 0.833 |

| *Kit 2* | | | | | |
|---|---|---|---|---|---|
| Non-pernicious stroke | N=23 | 0.040 | 0.050 | <0.0001 | 0.901 |
| Pernicious stroke | N=16 | 1.560 | 9.915 | | |

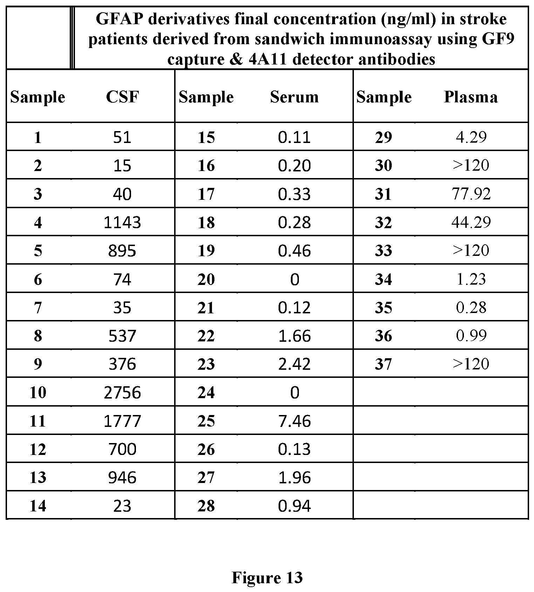

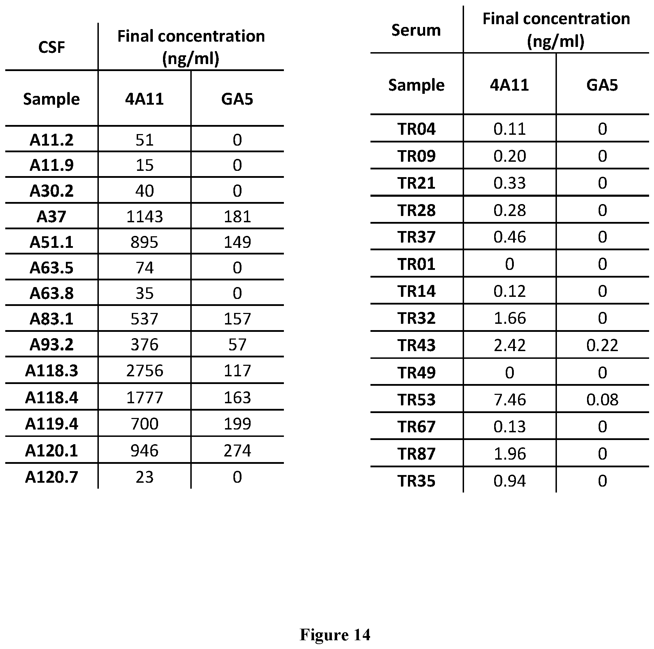

GFAP Kits 1 & 2 use different antibodies and differ in their sensitivities (Figure 1 and Table 2) - by detecting GFAP derivatives the sensitivity of the test is increased as shown by the P-value and AUC value. Kit 1 provides a sensitivity of 44% and specificity of 96% at a GFAP cut-off concentration of 0.160 ng/ml. Kit 2 provides a sensitivity of 75% and specificity of 100% at a GFAP cut-off concentration of 0.185 ng/ml. Figure 1 shows that using the antibodies that detect native GFAP only, over half of the non-pernicious stroke individuals have undetectable levels (upper graph of Figure 1), in contrast to total GFAP detection (lower graph of Figure 1). The finding that GFAP derivatives are present in the blood of patients undergoing HS, HT or IS fatal in amounts that enables a more sensitive assay compared to the detection of native GFAP alone was unexpected and enables a far more sensitive assay for stroke diagnosis. Antibody characterisation showed that anti-GFAP capture from Kit 2 (GF9) bound to the peptide fragment spotted at the DTR position 4 of the biochip. This corresponds to the spotted peptide ETRASERAEMME (SEQ ID NO: 1)(arrow of Figure 7, top-left image). Neither antibody in Kit 1 nor the detector antibody GF10 of Kit 2 bound to this sequence. The detector antibody of Kit 1 (GA-5) bound to the sequence TIPVQTFSNLQIRE (SEQ ID NO: 8). Results showed that the most sensitive antibody pairing for use in an immunoassay method was the combination of GF9 and 4A11 antibodies (see Figure 13). This assay was of superior sensitivity than when using a GF9 capture antibody with a GA-5 detector antibody which may suggest that GFAP calpain digest products were not being detected or were not as efficiently detected when the GA-5 detector was used (see Figure 14). Thus, although an improved immunoassay based method to be used in the diagnosis of stroke can be achieved through detecting native GFAP and GFAP derivatives using various antibody capture-detector combinations, an immunoassay of increased sensitivity can be obtained using a capture-detector antibody combination in which each antibody binds discrete amino acid sequences within native GFAP (isoforms 1,2 or 3) residing within the range of 60-390 amino acids.

### References

Jickling GC et al (2014). Journal of Cerebral Blood Flow & Metabolism, 34: 185-199.

Moeton M. et al (2016). Cellular and Molecular Life Sciences, 73: 4101-4120.

## Claims

1. An immunoanalytical method of diagnosing, or supporting the diagnosis of, stroke in a subject comprising measuring the amount of a GFAP derivative comprising the sequence ETRASERAEMME present in a sample which has been taken from the subject, the method incorporating an antibody which binds to an epitope of the GFAP derivative within the sequence ETRASERAEMME, wherein an amount of GFAP derivative in the sample bound by the antibody which is greater than the amount of the GFAP in a control subject is indicative of stroke.

2. The method of claim 1 in which the stroke is haemorrhagic stroke, haemorrhagic transformation or fatal ischaemic stroke.

3. The method of any of the preceding claims in which the GFAP control level is one that has been measured in a healthy patient or a healthy population of patients, or has been derived from a population of patients categorised as one or more of TIA, stroke mimic or ischaemic stroke.

4. The use of a GFAP derivative comprising the sequence ETRASERAEMME as a marker of haemorrhagic stroke, haemorrhagic transformation or fatal ischaemic stroke in an in-vitro diagnostic assay, wherein the sequence ETRASERAEMME is the target of an antibody in the diagnostic assay.

5. The use of claim 4 in which the GFAP derivative has a molecular weight of about 44 kDa and preferably wherein the epitope within the sequence ETRASERAEMMEcomprises at least one amino acid within the sequence SERA of the GFAP derivative.

6. An antibody which binds to an epitope of GFAP and the epitope is incorporated in the sequence ETRASERAEMME.

7. The antibody of claim 6 in which the epitope to which the antibody binds is within the sequence ETRASERAEMME and comprises at least one amino acid from the sequence SERA

8. The antibody of claims 6 and 7 in which GFAP is a GFAP derivative of molecular weight less than native GFAP without post translational modification and has a molecular weight of about 44 kDa.

9. The antibody of claims 6 to 8 which is attached to or adsorbed on the surface of a solid-state device, preferably wherein the solid-state device is a biochip.

10. The use of an antibody of any of claims 6 to 9 for *in-vitro* diagnosis of stroke.

11. The use of claim 12 in which the stroke is haemorrhagic stroke, haemorrhagic transformation or fatal ischaemic stroke.

12. A kit which incorporates an antibody of any of claims 6 to 9, preferably wherein the kit incorporates a further antibody that binds to an epitope of a GFAP species with the proviso that it does not bind to an amino acid within the sequence ETRASERAEMME.

13. Use of a capture and detector antibody each binding to a discrete epitope of non-overlapping amino acid residues comprised within the amino acid sequence 60 to 383, wherein the amino acid sequence 60-383 is a fragment of any one of SEQ ID NOs: 9 to 11 in an immunoassay method for diagnosing or supporting the diagnosis of stroke, wherein one of the capture and detector antibodies binds to an epitope of GFAP within the sequence ETRASERAEMME.

14. The use of claim 13 in which one of SEQ ID NO: 1 and SEQ ID NO: 3 is bound by the capture antibody (GF9) and the other is bound by the detector antibody. (4A11)

15. The use of claims 13 and 14 in diagnosing or supporting the diagnosis of haemorrhagic stroke, haemorrhagic transformation or fatal ischaemic stroke.

## Patentansprüche

1. Immunanalytisches Verfahren zum Diagnostizieren oder Unterstützen der Diagnose von Schlaganfall bei einem Subjekt, das Messen der Menge eines GFAP-Derivats umfasst, welches die Sequenz ETRASERAEMME umfasst, die in einer Probe vorhanden ist, die dem Subjekt entnommen wurde, wobei das Verfahren einen Antikörper beinhaltet, der an ein Epitop des GFAP-Derivats innerhalb der Sequenz ETRASERAEMME bindet, wobei eine vom Antikörper gebundene Menge des GFAP-Derivats in der Probe, die größer ist als die Menge des GFAP bei einem Kontrollsubjekt, auf Schlaganfall hinweist.

2. Verfahren nach Anspruch 1, wobei der Schlaganfall ein hämorrhagischer Schlaganfall, eine hämorrhagische Transformation oder ein tödlicher ischämischer Schlaganfall ist.

3. Verfahren nach einem der vorstehenden Ansprüche, wobei das GFAP-Kontrollniveau eines ist, das bei einem gesunden Patienten oder einer gesunden Patientenpopulation gemessen wurde oder aus einer Patientenpopulation abgeleitet wurde, die als eines oder mehrere von TIA, Schlaganfall-Imitator oder ischämischer Schlaganfall kategorisiert ist.

4. Verwendung eines GFAP-Derivats, das die Sequenz ETRASERAEMME umfasst, als Marker für hämorrhagischen Schlaganfall, hämorrhagische Transformation oder tödlichen ischämischen Schlaganfall in einem In-vitro-Diagnosetest, wobei die Sequenz ETRASERAEMME das Ziel eines Antikörpers im Diagnosetest ist.

5. Verwendung nach Anspruch 4, wobei das GFAP-Derivat ein Molekulargewicht von etwa 44 kDa aufweist und bevorzugt wobei das Epitop innerhalb der Sequenz ETRASERAEMME mindestens eine Aminosäure innerhalb der Sequenz SERA des GFAP-Derivats umfasst.

6. Antikörper, der an ein Epitop von GFAP bindet und das Epitop in der Sequenz ETRASERAEMME beinhaltet ist.

7. Antikörper nach Anspruch 6, wobei das Epitop, an das der Antikörper bindet, sich innerhalb der Sequenz ETRASERAEMME befindet und mindestens eine Aminosäure aus der Sequenz SERA umfasst.

8. Antikörper nach den Ansprüchen 6 und 7, wobei GFAP ein GFAP-Derivat mit einem Molekulargewicht von kleiner als nativem GFAP ohne posttranslationale Modifikation ist und ein Molekulargewicht von etwa 44 kDa aufweist.

9. Antikörper nach den Ansprüchen 6 bis 8, der an die Oberfläche einer Festkörpervorrichtung gebunden oder adsorbiert ist, bevorzugt wobei die Festkörpervorrichtung ein Biochip ist.

10. Verwendung eines Antikörpers nach einem der Ansprüche 6 bis 9 zur *In-vitro-*Diagnose von Schlaganfall.

11. Verwendung nach Anspruch 12, wobei der Schlaganfall ein hämorrhagischer Schlaganfall, eine hämorrhagische Transformation oder ein tödlicher ischämischer Schlaganfall ist.

12. Set, das einen Antikörper nach einem der Ansprüche 6 bis 9 beinhaltet, wobei das Set bevorzugt einen weiteren Antikörper beinhaltet, der an ein Epitop einer GFAP-Spezies bindet, mit der Maßgabe, dass er nicht an eine Aminosäure innerhalb der Sequenz ETRASERAEMME bindet.

13. Verwendung eines Fänger- und Detektorantikörpers, die jeweils an ein diskretes Epitop nicht überlappender Aminosäurereste binden, die innerhalb der Aminosäuresequenz 60 bis 383 umfasst sind, wobei die Aminosäuresequenz 60-383 ein Fragment einer von SEQ ID Nr. 9 bis 11 ist, in einem Immuntestverfahren zum Diagnostizieren oder Unterstützen der Diagnose von Schlaganfall, wobei einer des Fänger- und des Detektorantikörpers an ein Epitop von GFAP innerhalb der Sequenz ETRASERAEMME bindet.

14. Verwendung nach Anspruch 13, wobei eine von SEQ ID Nr. 1 und SEQ ID Nr. 3 durch den Fängerantikörper (GF9) gebunden wird, und die andere durch den Detektorantikörper (4A11) gebunden wird.

15. Verwendung nach den Ansprüchen 13 und 14 beim Diagnostizieren oder Unterstützen der Diagnose von hämorrhagischem Schlaganfall, hämorrhagischer Transformation oder tödlichem ischämischem Schlaganfall.

## Revendications

1. Procédé immunoanalytique pour diagnostiquer ou étayer le diagnostic d'un accident vasculaire cérébral chez un sujet, comprenant la mesure de la quantité d'un dérivé de GFAP comprenant la séquence ETRASERAEMME présente dans un échantillon qui a été prélevé sur le sujet, le procédé incorporant un anticorps qui se lie à un épitope du dérivé de GFAP dans la séquence ETRASERAEMME, dans lequel une quantité de dérivé de GFAP dans l'échantillon lié par l'anticorps qui est supérieure à la quantité de GFAP chez un sujet témoin indique un accident vasculaire cérébral.

2. Procédé selon la revendication 1, dans lequel l'accident vasculaire cérébral est un accident vasculaire cérébral hémorragique, une transformation hémorragique ou un accident vasculaire cérébral ischémique mortel.

3. Procédé selon l'une quelconque des revendications précédentes, dans lequel le niveau de contrôle de GFAP est celui qui a été mesuré chez un patient sain ou une population saine de patients, ou a été obtenu d'une population de patients catégorisés en un ou plusieurs parmi AIT, faux accident vasculaire cérébral ou accident vasculaire cérébral ischémique.

4. Utilisation d'un dérivé de GFAP comprenant la séquence ETRASERAEMME comme marqueur d'un accident vasculaire cérébral hémorragique, d'une transformation hémorragique ou d'un accident vasculaire cérébral ischémique mortel dans un test de diagnostic in vitro, dans laquelle la séquence ETRASERAEMME est la cible d'un anticorps dans le test de diagnostic.

5. Utilisation selon la revendication 4, dans laquelle le dérivé de GFAP présente une masse moléculaire d'environ 44 kDa et, de préférence, dans laquelle l'épitope dans la séquence ETRASERAEMME comprend au moins un acide aminé dans la séquence SERA du dérivé de GFAP.

6. Anticorps qui se lie à un épitope de GFAP et l'épitope est incorporé dans la séquence ETRASERAEMME.

7. Anticorps selon la revendication 6, dans lequel l'épitope auquel l'anticorps se lie est dans la séquence ETRASERAEMME et comprend au moins un acide aminé de la séquence SERA.

8. Anticorps selon les revendications 6 et 7, dans lequel GFAP est un dérivé de GFAP de masse moléculaire inférieure à la GFAP native sans modification post-traductionnelle et présente une masse moléculaire d'environ 44 kDa.

9. Anticorps selon les revendications 6 à 8, qui est fixé à ou adsorbé sur la surface d'un dispositif à semi-conducteurs, de préférence dans lequel le dispositif à semi-conducteurs est une biopuce.

10. Utilisation d'un anticorps selon l'une quelconque des revendications 6 à 9 pour le diagnostic *in vitro* d'un accident vasculaire cérébral.

11. Utilisation selon la revendication 12, dans laquelle l'accident vasculaire cérébral est un accident vasculaire cérébral hémorragique, une transformation hémorragique ou un accident vasculaire cérébral ischémique mortel.

12. Kit qui incorpore un anticorps selon l'une quelconque des revendications 6 à 9, de préférence dans lequel le kit incorpore un autre anticorps qui se lie à un épitope d'une espèce GFAP à condition qu'il ne se lie pas à un acide aminé dans la séquence ETRASERAEMME.

13. Utilisation d'anticorps de capture et de détection se liant chacun à un épitope discret de résidus d'acides aminés non chevauchants compris dans la séquence d'acides aminés 60 à 383, dans laquelle la séquence d'acides aminés 60-383 est un fragment de l'une quelconque des SEQ ID NO : 9 à 11 dans un procédé de dosage immunologique pour diagnostiquer ou étayer le diagnostic d'accident vasculaire cérébral, dans laquelle l'un des anticorps de capture et de détection se lie à un épitope de GFAP dans la séquence ETRASERAEMME.

14. Utilisation selon la revendication 13, dans laquelle l'une de SEQ ID NO : 1 et SEQ ID NO : 3 est liée par l'anticorps de capture (GF9) et l'autre est liée par l'anticorps détecteur. (4A11)

15. Utilisation des revendications 13 et 14 pour diagnostiquer ou étayer le diagnostic d'un accident vasculaire cérébral hémorragique, d'une transformation hémorragique ou d'un accident vasculaire cérébral ischémique mortel.
